(19) Europäisches Patentamt / European Patent Office / Office européen des brevets

(11) **EP 3 650 843 B1**

(12) **EUROPEAN PATENT SPECIFICATION**

(45) Date of publication and mention
of the grant of the patent:
**19.01.2022 Bulletin 2022/03**

(21) Application number: **18205002.1**

(22) Date of filing: **07.11.2018**

(51) International Patent Classification (IPC):
***G01N 21/84*** *(2006.01)*

(52) Cooperative Patent Classification (CPC):
**G01N 21/8483**

(54) **METHODS AND DEVICES FOR PERFORMING AN ANALYTICAL MEASUREMENT**

VERFAHREN UND VORRICHTUNGEN ZUR DURCHFÜHRUNG EINER ANALYTISCHEN MESSUNG

PROCÉDÉS ET DISPOSITIFS POUR EFFECTUER UNE MESURE ANALYTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA TN**

(43) Date of publication of application:
**13.05.2020 Bulletin 2020/20**

(73) Proprietors:
• **Roche Diabetes Care GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **Berg, Max
68305 Mannheim (DE)**
• **Hailer, Fredrik
68305 Mannheim (DE)**
• **Limburg, Bernd
68305 Mannheim (DE)**

(74) Representative: **Altmann Stößel Dick
Patentanwälte PartG mbB
Theodor-Heuss-Anlage 2
68165 Mannheim (DE)**

(56) References cited:
EP-A1- 2 916 117    WO-A1-2017/059103
DE-A1-102016 202 428    US-A1- 2012 329 170
US-A1- 2013 267 032    US-A1- 2015 233 898
US-A1- 2017 098 137

**Description**

Technical Field

The present application refers to a method of performing an analytical measurement based on a color formation reaction in an optical test strip by using a mobile device having a camera. The invention further relates to computer program with program means for executing the method according to the invention. Further, the invention refers to a mobile device and a kit for performing an analytical measurement. Methods, computer programs, mobile devices and kits according to the present invention may be used in medical diagnostics, in order to for example qualitatively or quantitatively detect one or more analytes in one or more body fluids. Other fields of application of the present invention, however, are feasible.

Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, EP 0 821 234 A2 describes a diagnostic test carrier for the determination of an analyte from whole blood with the aid of a reagent system contained in the carrier and a method for the determination of an analyte from whole blood with the aid of the diagnostic test carrier. The diagnostic test carrier includes a color forming reagent. The test field has a sample application side to which the blood sample is delivered and a detection side where an optically detectable change occurs as a result of the reaction of the analyte with the reagent system. Furthermore, the test field is designed so that the erythrocytes contained in the sample do not reach the detection side. In addition, the test field comprises a transparent film and a first and a second superposed film layer applied thereto, wherein the first layer on the transparent film is substantially less light-scattering in the wet state than the overlying second layer.

With regard to the test chemicals comprised in test elements, reference may be made e.g. to J. Hoenes et al. : The Technology Behind Glucose Meters: Test Strips, Diabetes Technology & Therapeutics, Volume 10, Supplement 1, 2008, S-10 to S-26. Other types of test chemistry are possible and may be used for performing the present invention.

Typically, one or more optically detectable changes in the test chemical are monitored, in order to derive the concentration of the at least one analyte to be detected from these changes. In general, variable lighting conditions need to be taken into account. Thus, as an example, EP 2 916 117 A1 discloses color quantification of chemical test pads and titration of analytes which can be performed under different lighting conditions. In one embodiment, the lighting condition under which a digital image is captured is estimated and utilized to select a set of reference colors for determining the titration. In another embodiment, a plurality of comparisons is made under different lighting conditions and the result having the highest confidence level is selected for determining the titration.

Further, WO 2014/037462 A1 discloses a method and a device for determining sample application on an analytical test element in a photometric reflectance measuring device specifically for glucose measurements, where the following measures are proposed: providing a disposable test element for application of a body fluid sample, taking a sequence of reflectance readings from the test element, monitoring a change of the reflectance readings with respect to a sample application condition, adjusting the sample application condition in accordance with a drift correction. A value for drift correction of the reflectance readings caused by ambient measurement conditions, specifically, humidity, temperature, or UV radiation can be considered in a predefined signal decrease or in a predefined signal threshold for adjusting the sample application condition.

For simple and reliable control of the suitability for use of analytical elements, EP 1 189 064 A1 proposes to calculate the variance of the quotient from a control value and a first standard reference value with respect to a first reference quotient which is from a control reference value and the first standard reference value.

EP 0 953 149 B1 discloses a method of analysis of image data from a chemical diagnostic assay which assay generates an image result on a substrate. The method comprising the steps of: i) obtaining a said image result on a substrate; and ii) imaging the image result with an image acquisition device to generate digital colour image data corresponding to the colour composition and distribution of the image result; and iii) using data processing means, applying to the digital colour image data a stored relationship between the colour composition of the image result and assay calibration data to generate a quantified result for said assay. Further, said assay calibration data comprises a set of calibration values for combinations of at least two selected colour parameters; and said quantified result is generated by interpolation between at least two of said calibration values.

The documents US 2015/0233899 A1, US 2017/0098137 A1 and US 2013/0267032 A1 teach methods and systems for reading test strips by using mobile devices.

The document WO 2017/059103 A1 teaches a method of reading test strips by capturing a first image of an undeveloped test strip and by capturing a second image after a test sample has been applied to the test step, whereby the second image is compared to the first image by subtracting the pre image from the post image to produce a background corrected image.

Besides using customized detectors which are specifically developed for the purpose of optically detecting changes in the test chemistry comprised by corresponding test elements, recent developments aim at using widely available devices such as smartphones. However, when using consumer-electronics having a camera, such as smartphones, individual technical and optical properties may have to be taken into account, since there is a vast number of cameras available on the market, which may have an impact on the determination of the analyte concentration.

Thus, despite the advantages involved in using consumer-electronics having a camera for the purpose of detecting an analyte in a sample or evaluating analytical measurements, several technical challenges remain. In general, color representation in camera systems is adapted to provide images which are optimized in terms of human color perception, e.g. by internal post-processing of the raw data captured by the camera. However, such post-processing due to human color perception may not be ideal when aiming at accurately determining analyte concentrations in the sample.

Problem to be solved

It is therefore desirable to provide methods and devices which address the above-mentioned technical challenges of analytical measurements using mobile devices such as consumer-electronics mobile devices, specifically multipurpose mobile devices which are not dedicated to analytical measurements such as smartphones or tablet computers. Specifically, methods, computer programs and devices shall be proposed which are widely applicable to available mobile devices and which are suited to increase measurement accuracy and improve reliability while allowing convenient handling for the user.

Summary

This problem is addressed by methods, computer programs and devices with the features of the independent claims 1, 9, 10 and 13. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method, as defined in claim 1, of performing an analytical measurement based on a color formation reaction in an optical test strip by using a mobile device having a camera is disclosed. The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

In general, the method comprises the following steps:

a) providing an optical test strip having a test field without having a sample applied thereto;
b) capturing at least one first image of at least part of the test field of the optical test strip without having a sample applied thereto by using the camera with at least one image acquisition setting, specifically with a set of acquisition settings of the camera;
c) applying a sample, specifically a drop, of bodily fluid to the test field of the optical test strip;
d) waiting for a predetermined minimum amount of time;
e) capturing at least one second image of at least part of the test field of the optical test strip having the sample of bodily fluid applied thereto by using the camera with the one or more image acquisition settings of the camera, wherein the image acquisition settings of the camera are the same image acquisition settings of the camera as used in step b); and
f) determining an analytical measurement result value by using the first and the second image of the optical test field of the optical test strip, specifically by comparing the at least two images.

The term "analytical measurement" as used herein is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample. For example, the sample may comprise a bodily fluid, such as blood, interstitial fluid, urine, saliva or other types of body fluids. The result of the analytical measurement, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value may be determined by the analytical measurement. The term "analytical measurement result value" as used herein is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value. The at least one sample, specifically, may be or may comprise at least one bodily fluid, such as blood, interstitial fluid, urine, saliva or the like. Additionally or alternatively, however, other types of samples may be used, such as water.

The analytical measurement, specifically, may be an analytical measurement including a change of at least one optical property of an optical test strip, which change may be measured or determined visually by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction.

The term "optical test strip" as used herein is a broad term and is to be given its ordinary and customary meaning to a person of ordinary skill in the art and is not to be limited to a special or customized meaning. The term specifically may refer, without limitation, to an arbitrary element or device configured for performing a color-change detection reaction. The optical test strip may also be referred to as test strip or test element, wherein all three terms may refer to the same element. The optical test strip may particularly have a test field containing at least one test chemical for detecting at least one analyte. The optical test strip, as an example, may comprise at least one substrate, such as at least one carrier, with the at least one test field applied thereto or integrated therein. In particular, the optical test strip may further comprise at least one white area, such as a white field, specifically in a proximity to the test field, for example enclosing or surrounding the test field. The white are may be a separate field independently arranged on the substrate or carrier. However, additionally or alternatively, the substrate or carrier itself may be or may comprise the white area. As an example, the at least one carrier may be strip-shaped, thereby rendering the test element a test strip. These test strips are generally widely in use and available. One test strip may carry a single test field or a plurality of test fields having identical or different test chemicals comprised therein.

As further used herein, the term "test field" is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. a larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

Both steps b) and e) comprise capturing at least one image by using the camera, wherein the camera is used with the at least one image acquisition setting. As used herein, the term "image acquisition setting" is a broad term and is to be given its ordinary and customary meaning to a person of ordinary skill in the art and is not to be limited to a special or customized meaning. The term specifically may refer, without limitation, to an arbitrary configuration or adjustment of an object or element, specifically of an imaging device, e.g. of a camera, when the object or element is used for capturing at least one image or picture. In particular, image acquisition settings may be configurations of the camera, such as the camera of the mobile device, when capturing the at least one image. For example, the image acquisition setting may be one or more of a shutter speed, an exposure time, a lens focus, a color adjustment, a color saturation, a pixel correction, a noise reduction or the like. Thus, when a camera is used for capturing at least one image, the image acquisition settings of the camera may also simply be referred to as image acquisition settings.

In particular, the image acquisition settings may for example be automatically chosen by the mobile device, e. g. by the mobile device having the camera, used for capturing the image. Thus, as an example, the mobile device having the camera, e.g. a smartphone or a smartphone camera system, may automatically choose or set the image acquisition settings according to an ambient situation, e.g. according to an ambient lighting. Additionally or alternatively, one, more than one or even all of the image acquisition settings may be defined or set manually or via a predefined algorithm.

Step b) further comprises locking the one or more image acquisition settings of the camera. In particular, the one or more image acquisition settings of the camera remain locked at least until step e) has been carried out, specifically at least until the second image of the test field is captured. As used herein, the term "locking" is a broad term and is to be given its ordinary and customary meaning to a person of ordinary skill in the art and is not to be limited to a special or customized meaning. The term specifically may, without limitation, refer to a process of keeping an arbitrary configuration consistent, e.g. unchanged and/or unvaried. As an example, the locked configuration may remain constant for a period of time, specifically for a time between at least two events. Particularly, when locking a configuration, starting at a first event the configuration or setting may for example remain unchanged until at least one second event. Thus, the locking of the one or more image acquisition settings comprises keeping the image acquisition settings constant or unchanged for a period of time, specifically for the duration between carrying out step b) of the method until step e) of the method has been carried out.

Specifically, step b) may further comprise deriving at least one parameter pertaining to the one or more image acquisition settings. For example, the parameter pertaining to the one or more image acquisition settings may be or may comprise at least one numerical value referring to the one or more image acquisition settings.

In particular, the parameter pertaining to the one or more image acquisition settings may for example be stored in a data base, specifically in a memory of the mobile device and/or the camera. Thus, the parameter pertaining to the one or more image acquisition settings may be stored within the mobile device, specifically within the memory of the mobile device having the camera, used for capturing the images in steps b) and e). Additionally or alternatively, however, the parameter pertaining to the one or more image acquisition settings may also be stored in an external memory or data base, for example in a cloud. Specifically, the data base may be stored in a data storage device, such as for example in a memory, such as a volatile or non-volatile memory, e.g. in a memory of the mobile device, on an external hard disk or hard drive, on an external computer or computer network, on a solid-state-drive or in a cloud.

As an example, the at least one parameter pertaining to the one or more image acquisition settings may be selected from the group consisting of: a shutter speed, specifically an exposure time; a focus distance; a color adjustment, such as a color saturation; a pixel correction, specifically a noise reduction.

Further, at least one of the at least one parameters pertaining to the one or more image acquisition settings may be configured to be corrected or set by at least one algorithm, for example in order to prevent overexposure, specifically of white areas within the image. As an example, the at least one algorithm may be configured for correcting the at least one of the at least one parameters in order to ensure a minimum amount of image quality, specifically of the images captured in steps b) and e). In particular, the algorithm may for example be stored and/or run on the mobile device. Specifically, the algorithm may be stored in a memory of the mobile device and/or may be loadable into a processor of the mobile device. Additionally or alternatively, the algorithm may be run on the mobile device, for example on a processor of the mobile device.

Step c) may specifically comprise one or both of:

- prompting a user to apply a sample, specifically a drop, of bodily fluid to the test field of the optical test strip; or
- prompting the user to confirm application of the sample of bodily fluid to the test field of the optical test strip.

Further, step d) may comprise waiting for at least 5 s. Thus, the predetermined minimum amount of time may for example be at least 5 s.

Further, step f) may comprise comparing the at least two images captured in steps b) and e), respectively. In particular, when comparing the at least two images at least one of a color difference and an intensity difference may be detected. Thus, step f) may particularly comprise detecting one or both of the color difference and the intensity difference, for example by comparing the images, e.g. the pixels of the images, captured in steps b) and e).

In particular, step f) comprises determining a relative measurement value $R_{rel}$ from one or both of a color or an intensity of the test field of the first image $I_{TF\_1}$ and a color and/or an intensity of the test field of the second image $I_{TF\_2}$ by using the following function:

$$R_{rel} = \frac{I_{TF\_2}}{I_{TF\_1}}. \qquad (1)$$

Specifically, the function may further comprise a proportionality factor.

Additionally or alternatively, step f) comprises determining a twice relative measurement value $R_{twice\_rel}$ from a color and/or an intensity of the test field of the first image $I_{TF\_1}$, a color and/or an intensity of the test field of the second image $I_{TF\_2}$, a color and/or an intensity of a reference area, e.g. a white area, on the optical test strip of the first image $I_{WF\_1}$ and a color and/or an intensity of the same reference area on the optical test strip of the second image $I_{WF\_2}$, by using the following functions:

$$R_{twice\_rel} = \frac{I_{TF\_2} / I_{WF\_2}}{I_{TF\_1} / I_{WF\_1}}. \qquad (2)$$

Specifically, the function may further comprise a proportionality factor.

The method may further comprise step g) evaluating at least one ambient lighting condition. Specifically, an illumination, for example an illumination of the test field, may be evaluated in step g). In particular, step g) may for example be performed at least once at one or more of the following points in time:

- between step a) and step b),
- between step b) and step c),

- between steps e) and f), or
- after step f).

In particular, the at least one ambient lighting condition may specifically be evaluated at least once before performing step b) and for example after performing step a). Additionally or alternatively, step g) may for example be performed at least once before performing step e).

Specifically, step g) may further comprise adapting the at least one ambient lighting condition by turning on or off at least one illumination source of the mobile device. The term "illumination source" as used herein is a broad term and is to be given its ordinary and customary meaning to a person of ordinary skill in the art and is not to be limited to a special or customized meaning. The term specifically may, without limitation, refer to an arbitrary device adapted to generate light for illuminating an object. For example, the illumination source of the mobile device may comprise at least one light-emitting diode integrated into the mobile device. In particular, the illumination source may be a backlighting of the mobile device. The mobile device may comprise further illumination devices such as at least one illumination source illuminating the display and/or the display may itself be designed as further illumination source itself. Thus, as an example, the at least one illumination source may be turned on in order to lighten or brighten the at least one ambient lighting condition. In particular, the at least one illumination source may be turned on when generating light, e.g. generating light from the at least one light-emitting diode or backlight of the mobile device, is required. The at least one illumination source may be turned off when no light generation from the illumination source is required.

In a further aspect, a computer program, as defined in claim 1, including computer-executable instructions for performing the method when the computer program is executed on a computer or computer network is disclosed. The computer program is configured for performing the method of performing an analytical measurement based on a color formation reaction as described above or as will be described in further detail below, when the computer program is executed on the computer or computer network, specifically on a processor of a mobile device. The computer program may specifically be configured for performing at least steps b), d), e), f) and optionally steps c) and/or g) of the method of performing an analytical measurement.

For possible definitions of terms and possible embodiments, reference may be made to the description given above or as described in further detail below.

Specifically, the computer program may be stored on a computer-readable data carrier.

Thus, further disclosed and proposed herein is a data carrier having a data structure stored thereon, which, after loading into a computer or computer network, such as into a working memory or main memory of the computer or computer network, may execute the method according to one or more of the embodiments disclosed herein.

In a further aspect, a mobile device, as defined in claim 10, for performing an analytical measurement is disclosed.

The mobile device has at least one camera. Further, the mobile device is configured for performing at least steps b), d), e) and f) of the method of performing an analytical measurement as described above or as will be described in further detail below.

Again, for possible definitions of terms and possible embodiments, reference may be made to the description given above or as described in further detail below.

Further, the mobile device may comprise at least one illumination source configured for adapting at least one ambient lighting condition.

In particular, the mobile device may comprise at least one processor being programmed for controlling at least one of steps b), d), e), and f), and optionally steps c) and/or g). The term "processor" as used herein is a broad term and is to be given its ordinary and customary meaning to a person of ordinary skill in the art and is not to be limited to a special or customized meaning. The term specifically may refer, without limitation, to an arbitrary electronic circuit configured for operating on data. In particular, the processor may be configured for performing operations on at least one data base, such as on a memory, e.g. on a memory of the mobile device. As an example, the processor may specifically be or may comprise an integrated circuit (IC), such as an application-specific integrated circuit (ASIC) or a field programmable gate array (FPGA).

In a further aspect, a kit, as defined in claim 13, for performing an analytical measurement is disclosed. The kit comprises:

- at least one mobile device as described above or as will be described in further detail below; and
- at least one optical test strip having at least one test field.

Again, for possible definitions of terms and possible embodiments, reference may be made to the description given above or as described in further detail below.

The term "kit" as used herein is a broad term and is to be given its ordinary and customary meaning to a person of ordinary skill in the art and is not to be limited to a special or customized meaning. The term specifically may refer, without limitation, to an assembly of a plurality of components, wherein the components each may function and may be handled independently from each other, wherein the components of the kit may interact to perform a common function.

The methods, computer programs and devices as disclosed herein may provide a large number of advantages over known methods, computer programs and devices. Thus, in particular, the present invention may provide for an improved reliability and accuracy of the analytical measurement compared to methods, computer programs and devices known from the art. Specifically, the reliability and accuracy may be improved by taking into account image processing steps of the mobile device, specifically any image processing steps performed by software, e.g. by an app, on the mobile device.

Further, the present invention may simplify the process of performing the analytical measurement for a user. Specifically, the user may be able to perform the analytical measurement using a widely available mobile device, such as a smartphone, instead of a customized detector. In particular, the present invention may be suitable for a vast number of mobile devices, such as a great variety of mobile phones or smartphones, specifically because the present invention may for example be technically compatible with a majority of mobile devices, such that a technical implementation and/or use may be possible on a large number of mobile devices, such as mobile devices running at least one operating system, such as at least one operating system selected from the consisting of Android, Windows, iOS or Linux. Other operating systems may be feasible.

In addition, for the present invention requirements for the camera system of the mobile device may be reduced compared to methods, computer programs and devices known from the art. In particular, the requirements for absolute color measurement or detection may for example be reduced due to a relative measurement, specifically a twice relative measurement, which may be used when performing the analytical measurement of the present invention. Specifically, in the present invention analyte measurement by using a relative color measurement, e.g. a relative intensity measurement, may for example be proposed. In particular, a first image of the optical test strip, e.g. of the test element, may be captured before application of the sample, specifically before the sample is applied to the test field of the optical test strip. When capturing the first image, the image acquisition settings may for example be chosen automatically by the mobile device, e.g. by the mobile device having the camera, used for capturing the image. Thus, as an example, the mobile device having the camera, e.g. a smartphone or a smartphone camera system, may automatically choose the image acquisition settings according to an ambient situation, e.g. according to an ambient lighting, when capturing the image, wherein overexposure of , for example, relevant parts of the optical test strip, e.g. of a white field, may be repressed. Additionally or alternatively, the image acquisition settings may be chosen manually or via a predefined algorithm. Subsequently, as an example, a capturing of a second image of the test element, e.g. after the sample has been applied to the test field, may specifically be performed using the same image acquisition settings. Thus, the image acquisition settings chosen when capturing the first image may be locked and/or stored, for example within the mobile device, until capturing the second image. Such functionality of locking and/or storing the image acquisition settings may specifically be supported by a vast number of mobile devices, e.g. by a large number of smartphones.

Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments but only by the appended claims. . The embodiments are schematically depicted in the Figures. Therein, identical reference numbers in these Figures refer to identical or functionally comparable elements.

In the Figures:

Figure 1 shows a perspective view of an embodiment of a kit and a mobile device;

Figures 2A and 2B show flow charts of embodiments of a method for performing an analytical measurement; and

Figures 3A and 3B show embodiments of diagrams indicating color signal counts recognizable by a camera over time when performing an analyte measurement.

Detailed description of the embodiments

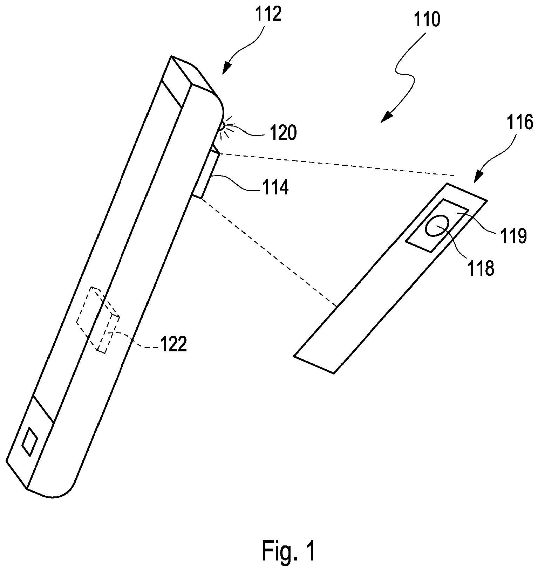

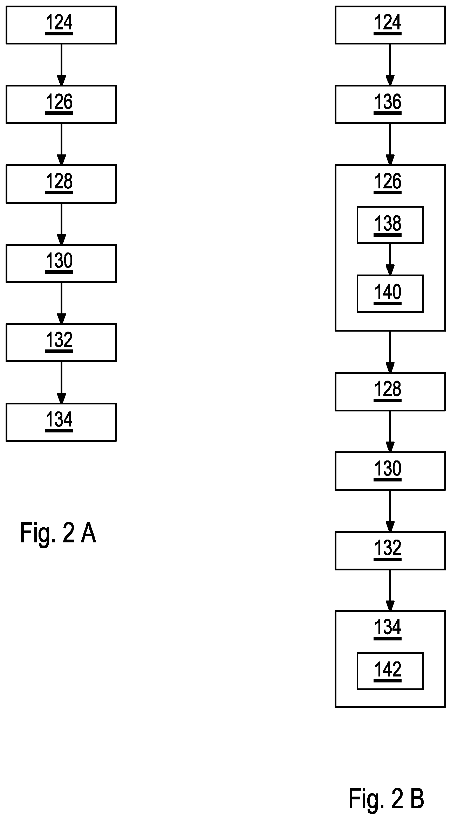

In Figure 1 a kit 110 for performing analytical measurement is shown in a perspective view. The kit 110 comprising at least one mobile device 112 having at least one camera 114, the kit further comprising at least one optical test strip 116 having at least one test field 118. The optical test strip 116 may further have at least one white area 119. The mobile device 112 is configured for performing a method of performing an analytical measurement, as for example shown in Figures 2A and 2B. As illustrated in Figure 1, the mobile device 112 may further comprise at least one illumination source 120. In particular, the mobile device 112 may comprise at least one processor 122 being programmed for controlling the steps of the method of performing an analytical measurement.

Figure 2A shows an embodiment of a flow chart of a method of performing an analytical measurement based on a color formation reaction in an optical test strip 116 by using a mobile device 112 having a camera 114. The method comprises the following steps:

a) (indicated with reference number 124) providing an optical test 116 strip having a test field 118 without having a sample applied thereto;

b) (indicated with reference number 126) capturing at least one first image of at least part of the test field 118 of the optical test strip 116 without having a sample applied thereto by using the camera 114 with at least one image acquisition setting of the camera 114, specifically with a set of acquisition settings of the camera 114;

c) (indicated with reference number 128) applying one or both of a sample, specifically a drop, of bodily fluid to the test field 118 of the optical test strip 116;

d) (indicated with reference number 130) waiting for a predetermined minimum amount of time;

e) (indicated with reference number 132) capturing at least one second image of at least part of the test field of the optical test strip having the sample of bodily fluid applied thereto by using the camera with the one or more image acquisition settings of the camera 114, wherein the image acquisition settings of the camera are the same image acquisition settings of the camera 114 as used in step b); and

f) (indicated with reference number 134) determining an analytical measurement result value by using the first and the second image of the optical test field of the optical test strip, specifically by comparing the at least two images.

The mobile device 112 is in particular configured for performing at least steps b), d), e) and f) of the method of performing an analytical measurement. In particular, the processor 122 may be programmed for controlling at least one of steps b), d), e) and f).

In Figure 2B, a different embodiment of a flow chart of the method of performing an analytical measurement is shown. The method may further comprise step g) (indicated with reference number 136) evaluating at least one ambient lighting condition. In particular, step g) may, as illustrated in the figure, be performed at least once between step a) and step b). However, additionally or alternatively, step g) may be performed between step b) and c) and/or between step e) and step f) and/or after step f). The method, specifically step b) of the method, further comprises at least one substep (indicated with reference number 138) of locking the one or more image acquisition settings of the camera 114. Specifically, the image acquisition settings or the set of image acquisition settings remain locked until step e) of the method has been carried out. In particular, the image acquisition settings may remain locked at least until the second image of the test field has been captured. Additionally or alternatively, step b) may comprise at least one substep (indicated with reference number 140) of deriving at least one parameter pertaining to the one or more image acquisition settings. Further, step f) comprises at least one substep (indicated with reference number 142) of comparing the at least two images captured in steps b) and e) respectively, in particular comparing the images with each other, wherein at least one of a color difference and an intensity difference may be detected. In step f) a relative measurement value $R_{rel}$ 144 is determined by using function (1) as described above. Additionally or alternatively, in step f) a twice relative measurement value $R_{twice\_rel}$ 146 is determined by using function (2) as described above.

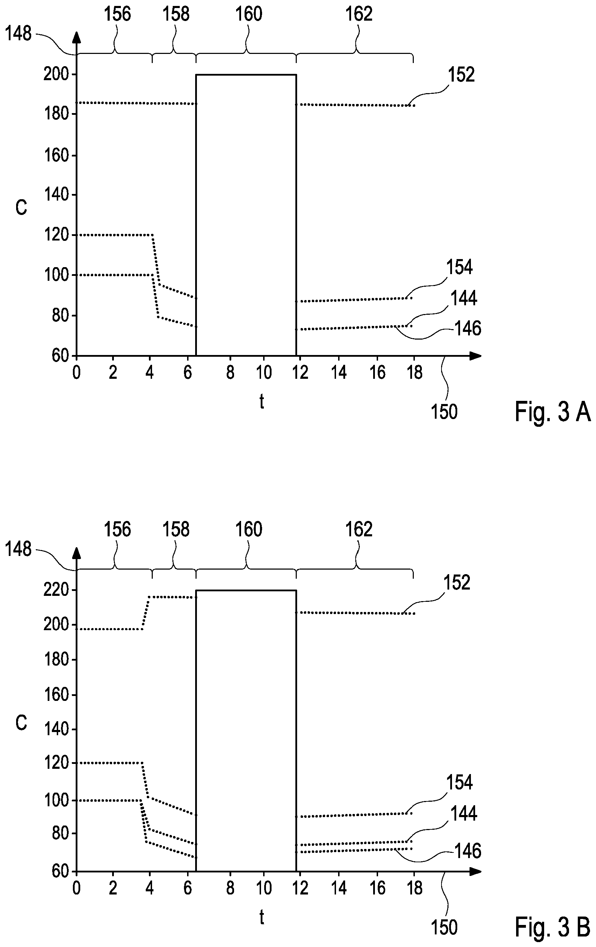

Figures 3A and 3B show embodiments of diagrams indicating color signal counts (C) recognizable by a camera 114, for example by the camera 114 of the mobile device 112, over time (t) when performing an analyte measurement. In particular, color signal counts for red color, e.g. R value 148, are shown over time measured in seconds 150. In the diagrams, measurement of samples with a blood glucose concentration of 50 mg/dl may be illustrated. Specifically, in the diagrams, color signal counts or R value for the white area 152 and the test field 154 of the optical test strip 116 are illustrated over time. Further, in the diagrams $R_{rel}$ 144 and $R_{twice\_rel}$ 146 are shown, wherein $R_{rel}$ 144 and $R_{twice\_rel}$ 146 may be determined by using functions (1) and (2) respectively.

As an example, in Figured 3A and 3B, performance of at least steps b), c), d) and e) of the method of performing an analytical measurement based on a color formation reaction may be illustrated. Thus, step b), in particular the capturing of the at least one first image, may be performed within a first time slot 156, wherein step c), specifically the application of a sample of bodily fluid to the test field 118, may be performed within a second time slot 158. Further, a third time slot 160 may as an example equal the predetermined minimum amount of time waited for in step d) of the method. Step e), in particular the capturing of the at least one second image, may be performed within a fourth time slot 162.

Specifically, in Figure 3A, ambient light condition is constant. Thus, for example, the R value of the white area 152 may show a constant course over time, e.g. over the duration of the measurement. As illustrated in Figure 3A, for constant ambient lighting conditions $R_{rel}$ 144 $\approx R_{twice\_rel}$ 146. However, in Figure 3B, ambient light condition changes. Thus, specifically the R value of the white area 152, illustrated in Figure 3B, may change in the course of the measurement. Further, as illustrated in Figure 3B, changing ambient light conditions may lead to $R_{rel}$ 144 $\neq R_{twice\_rel}$ 146. In particular, when determining $R_{rel}$ 144, as an example, the R value of the test field 154 after sample application may be set in relation to the R value of the test field 154 without having a sample applied thereto, wherein the R value of the white area 152 may not be taken into account when determining $R_{rel}$ 144. Thus, changing ambient lighting conditions indicated, for example, by the R value of the white area 152, specifically of the white field or reference field, may not be taken into account when determining $R_{rel}$ 144. However, the R value of the white area 152 may be used for determining $R_{twice\_rel}$ 146. Thus, when determining of $R_{twice\_rel}$ 146 ambient lighting conditions may be taken into account. In particular, in case $R_{twice\_rel}$ 146 is used in step f) of the method, the determined analytical measurement result value may for example even be independent of changes in ambient lighting during performing of the method.

List of reference numbers

**[0070]**

| | |
|---|---|
| 110 | kit |
| 112 | mobile device |
| 114 | camera |
| 116 | optical test strip |
| 118 | test field |
| 119 | white area |
| 120 | illumination source |
| 122 | processor |
| 124 | step a) |
| 126 | step b) |
| 128 | step c) |
| 130 | step d) |
| 132 | step e) |
| 134 | step f) |
| 136 | step g) |
| 138 | substep |
| 140 | substep |
| 142 | substep |
| 144 | $R_{rel}$ |
| 146 | $R_{twice\_rel}$ |
| 148 | R value |
| 150 | seconds |
| 152 | R value of white area |
| 154 | R value of test field |
| 156 | first time slot |
| 158 | second time slot |
| 160 | third time slot |
| 162 | fourth time slot |

**Claims**

1.  A method of performing an analytical measurement based on a color formation reaction in an optical test strip (116) by using a mobile device (112) having a camera (114), the method comprising:

    a) providing an optical test strip (116) having a test field (118) without having a sample applied thereto;
    b) capturing at least one first image of at least part of the test field (118) of the optical test strip (116) without having a sample applied thereto by using the camera (114) with at least one image acquisition setting of the camera (114);
    c) applying a sample of bodily fluid to the test field (118) of the optical test strip (116);
    d) waiting for a predetermined minimum amount of time;
    e) capturing at least one second image of at least part of the test field (118) of the optical test strip (116) having the sample of bodily fluid applied thereto by using the camera (114) with the one or more image acquisition settings of the camera (114), wherein the image acquisition settings of the camera (114) are the same image acquisition settings of the camera (114) as used in step b); and
    f) determining an analytical measurement result value by using the first and the second image of the optical test field (118) of the optical test strip (116),

wherein step b) further comprises locking the one or more image acquisition settings of the camera (114), wherein the one or more image acquisition settings of the camera (114) remain locked at least until step e) has been carried out, wherein step f) comprises comparing the at least two images captured in steps b) and e) respectively, wherein at least one of a color difference and an intensity difference is detected, **characterized in that** step f) comprises determining a relative measurement value $R_{rel}$ from one or both of a color or an intensity of the test field of the first image $I_{TF\_1}$ and a color and/or intensity of the test field of the second image $I_{TF\_2}$ by using the following function:

$$R_{rel} = \frac{I_{TF\_2}}{I_{TF\_1}},$$

and/or **in that** step f) comprises determining a twice relative measurement value $R_{twice\_rel}$ from a color and/or an intensity of the test field of the first image $I_{TF\_1}$, a color and/or an intensity of the test field of the second image $I_{TF\_2}$, a color and/or an intensity of a reference area on the optical test strip of the first image $I_{WF\_1}$ and a color and/or an intensity of the same reference area on the optical test strip of the second image $I_{WF\_2}$, by using the following functions:

$$R_{twice\_rel} = \frac{I_{TF\_2} / I_{WF\_2}}{I_{TF\_1} / I_{WF\_1}}.$$

2. The method according to the preceding claim, wherein step b) further comprises deriving at least one parameter pertaining to the one or more image acquisition settings.

3. The method according to the preceding claim, wherein the parameter pertaining to the one or more image acquisition settings is stored in a data base.

4. The method according to the preceding claim, wherein the at least one parameter pertaining to the one or more image acquisition settings is selected from the group consisting of: a shutter speed, specifically an exposure time; a focus distance; a color adjustment, such as a color saturation; a pixel correction, specifically a noise reduction.

5. The method according to the preceding claim, wherein at least one of the at least one parameters pertaining to the one or more image acquisition settings is configured to be corrected or set by at least one algorithm.

6. The method according to any one of the preceding claims, wherein step c) comprises one or both of:

   - prompting a user to apply the sample of bodily fluid to the test field (118) of the optical test strip (116); or
   - prompting the user to confirm application of the sample of bodily fluid to the test field (118) of the optical test strip (116).

7. The method according to any one of the preceding claims, wherein the method further comprises
   g) evaluating at least one ambient lighting condition,
   wherein step g) is performed at least once at one or more of the following points in time:

   - between step a) and step b),
   - between step b) and step c),
   - between steps e) and f), or
   - after step f).

8. The method according to the preceding claim, wherein step g) further comprises adapting the at least one ambient lighting condition by turning on or off at least one illumination source (120) of the mobile device (112).

9. A computer program including computer-executable instructions for performing the method according to any one of the preceding claims when the computer program is executed on a computer or computer network.

10. A mobile device (112) for performing an analytical measurement, the mobile device (112) having at least one camera (114), the mobile device being configured for performing at least steps b), d), e) and f) of the method of performing an analytical measurement according to any one of the preceding claims referring to a method of performing an analytical measurement.

11. The mobile device (112) according to the preceding claim, wherein the mobile device (112) further comprises at least one illumination source (120) configured for adapting at least one ambient lighting condition.

12. The mobile device (112) according to any one of the two preceding claims, wherein the mobile device (112) comprises at least one processor (122) being programmed for controlling at least one of steps b), d), e), and f), and optionally c) and/or g).

13. A kit (110) for performing an analytical measurement, the kit (110) comprising:

- at least one mobile device (112) according to any one of the preceding claims referring to a mobile device (112); and
- at least one optical test strip (116) having at least one test field (118).

**Patentansprüche**

1. Verfahren zum Durchführen einer analytischen Messung, die auf einer Farbbildungsreaktion basiert, in einem optischen Teststreifen (116) unter Verwendung einer mobilen Vorrichtung (112) mit einer Kamera (114), wobei das Verfahren umfasst:

a) Bereitstellen eines optischen Teststreifens (116) mit einem Testfeld (118) ohne darauf aufgebrachte Probe;
b) Erfassen mindestens eines ersten Bildes von mindestens einem Teil des Testfelds (118) des optischen Teststreifens (116) ohne darauf aufgebrachte Probe unter Verwendung der Kamera (114) mit mindestens einer Bildakquisitionseinstellung der Kamera (114);
c) Aufbringen einer Probe von Körperfluid auf das Testfeld (118) des optischen Teststreifens (116);
d) Warten für eine vorgegebene Mindestzeitdauer;
e) Erfassen von mindestens einem zweiten Bild von mindestens einem Teil des Testfelds (118) des optischen Teststreifens (116) mit der darauf aufgebrachten Probe des Körperfluids unter Verwendung der Kamera (114) mit der einen oder den mehreren Bildakquisitionseinstellungen der Kamera (114), wobei die Bildakquisitionseinstellungen der Kamera (114) die gleichen Bildakquisitionseinstellungen der Kamera (114) sind, wie sie in Schritt b) verwendet wurden; und
f) Ermitteln eines analytischen Messergebniswerts unter Verwendung des ersten und des zweiten Bilds des optischen Testfelds (118) des optischen Teststreifens (116),

wobei Schritt b) des Weiteren Sperren der einen oder mehreren Bildakquisitionseinstellungen der Kamera (114) umfasst, wobei die eine oder mehreren Bildakquisitionseinstellungen der Kamera (114) gesperrt bleiben, bis Schritt e) ausgeführt worden ist, wobei Schritt f) Vergleichen der mindestens zwei in den Schritten b) beziehungsweise e) erfassten Bilder umfasst, wobei mindestens eine von einer Farbdifferenz und einer Intensitätsdifferenz detektiert wird, **dadurch gekennzeichnet, dass** Schritt f) umfasst:

Ermitteln eines relativen Messwerts $R_{rel}$ aus einer oder beiden von einer Farbe oder einer Intensität des Testfelds des ersten Bilds $I_{TF\_1}$ und einer Farbe und/oder Intensität des Testfelds des zweiten Bilds $I_{TF2}$ unter Verwendung der folgenden Funktion:

$$R_{rel} = \frac{I_{TF\_2}}{I_{TF\_1}}$$

und/oder

dadurch, dass Schritt f) Ermitteln eines doppelten relativen Messwerts $R_{doppelt\ rel}$ aus einer Farbe und/oder einer Intensität des Testfelds des ersten Bilds $I_{TF\_1}$, einer Farbe und/oder einer Intensität des Testfelds des zweiten Bildes $I_{TF\_2}$, einer Farbe und/oder einer Intensität einer Referenzfläche auf dem optischen Teststreifen des ersten Bilds $I_{WF\_1}$ und einer Farbe und/oder einer Intensität der gleichen Referenzfläche auf dem optischen Teststreifen des zweiten Bilds I$_{WF\,2}$ unter Verwendung der folgenden Funktionen umfasst:

$$R_{doppelt\_rel} = \frac{I_{TF\_2}/I_{WF\_2}}{I_{TF\_1}/I_{WF\_1}}$$

2. Verfahren nach dem vorhergehenden Anspruch, wobei Schritt b) des Weiteren Ableiten mindestens eines Parameters umfasst, der die eine oder mehreren Bildakquisitionseinstellungen betrifft.

3. Verfahren nach dem vorhergehenden Anspruch, wobei der Parameter, der die eine oder mehreren Bildakquisitionseinstellungen betrifft, in einer Datenbank gespeichert ist.

4. Verfahren nach dem vorhergehenden Anspruch, wobei der mindestens eine Parameter, der die mindestens eine oder mehreren Bildakquisitionseinstellungen betrifft, ausgewählt ist aus der Gruppe bestehend aus: einer Verschlussgeschwindigkeit, insbesondere einer Belichtungszeit; einer Fokusentfernung; einer Farbanpassung, wie einer Farbsättigung; einer Pixelkorrektur, speziell einer Rauschreduktion.

5. Verfahren nach dem vorhergehenden Anspruch, wobei mindestens einer der mindestens einen Parameter, der/die die eine oder mehreren Bildakquisitionseinstellungen betrifft/betreffen, konfiguriert ist, um durch mindestens einen Algorithmus korrigiert oder festgelegt zu werden.

6. Verfahren nach einem der vorhergehenden Ansprüche, wobei Schritt c) ein oder beide der folgenden umfasst:

   - Auffordern eines Anwenders, die Probe des Körperfluids auf das Testfeld (118) des optischen Teststreifens (116) aufzubringen; oder
   - Auffordern des Anwenders, das Aufbringen der Probe des Körperfluids auf das Testfeld (118) des optischen Teststreifens (116) zu bestätigen.

7. Verfahren nach einem der vorhergehenden Ansprüche, wobei das Verfahren des Weiteren umfasst:
   g) Auswerten von mindestens einer Umgebungsbeleuchtungsbedingung,
   wobei Schritt g) mindestens einmal an einem oder mehreren der folgenden Zeitpunkte durchgeführt wird:

   - zwischen Schritt a) und Schritt b),
   - zwischen Schritt b) und Schritt c),
   - zwischen den Schritten e) und f) oder
   - nach Schritt f).

8. Verfahren nach dem vorhergehenden Anspruch, wobei Schritt g) des Weiteren Anpassen der mindestens einen Umgebungsbeleuchtungsbedingung durch Einschalten oder Ausschalten von mindestens einer Beleuchtungsquelle (120) der mobilen Vorrichtung (112) umfasst.

9. Computerprogramm, das computerausführbare Anweisungen einschließt, um das Verfahren gemäß einem der vorhergehenden Ansprüche durchzuführen, wenn das Computerprogramm auf einem Computer oder Computernetzwerk ausgeführt wird.

10. Mobile Vorrichtung (112) zur Durchführung einer analytischen Messung, wobei die mobile Vorrichtung (112) mindestens eine Kamera (114) aufweist, die mobile Vorrichtung konfiguriert ist, um mindestens die Schritte b), d), e) und f) des Verfahrens zur Durchführung der analytischen Messung nach einem der vorhergehenden Ansprüche durchzuführen, die sich auf ein Verfahren zur Durchführung einer analytischen Messung beziehen.

11. Mobile Vorrichtung (112) nach dem vorhergehenden Anspruch, wobei die mobile Vorrichtung (112) des Weiteren mindestens eine Beleuchtungsquelle (120) umfasst, die zum Anpassen mindestens einer Umgebungsbeleuchtungsbedingung konfiguriert ist.

12. Mobile Vorrichtung (112) nach einem der beiden vorhergehenden Ansprüche, wobei die mobile Vorrichtung (112) mindestens einen Prozessor (122) umfasst, der zum Steuern von mindestens einem der Schritte b), d), e) und f) und optional c) und/oder g) programmiert ist.

13. Kit (110) zum Durchführen einer analytischen Messung, wobei das Kit (110) umfasst:

- mindestens eine mobile Vorrichtung (112) nach einem der vorhergehenden Ansprüche, die sich auf eine mobile Vorrichtung (112) beziehen; und
- mindestens einen optischen Teststreifen (116) mit mindestens einem Testfeld (118).

**Revendications**

1. Procédé de réalisation d'une mesure analytique basée sur une réaction de formation de couleur dans une bandelette de test optique (116) en utilisant un dispositif mobile (112) ayant une caméra (114), le procédé comprenant :

a) la fourniture d'une bandelette de test optique (116) ayant un champ de test (118) sans qu'un échantillon y soit appliqué ;
b) la capture d'au moins une première image d'au moins une partie du champ de test (118) de la bandelette de test optique (116) sans qu'un échantillon y soit appliqué en utilisant la caméra (114) avec au moins un réglage d'acquisition d'image de la caméra (114) ;
c) l'application d'un échantillon de fluide corporel sur le champ de test (118) de la bandelette de test optique (116) ;
d) l'attente pendant une durée minimale prédéterminée,
e) la capture d'au moins une seconde image d'au moins une partie du champ de test (118) de la bandelette de test optique (116) sur laquelle est appliqué l'échantillon de fluide corporel en utilisant la caméra (114) avec un ou plusieurs réglages d'acquisition d'image de la caméra (114), les réglages d'acquisition d'image de la caméra (114) étant les mêmes réglages d'acquisition d'image de la caméra (114) que ceux utilisés à l'étape b) ; et
f) la détermination d'une valeur de résultat de mesure analytique en utilisant la première et la seconde image du champ de test optique (118) de la bandelette de test optique (116), l'étape b) comprenant en outre le verrouillage d'un ou plusieurs réglages d'acquisition d'image de la caméra (114), le ou les réglages d'acquisition d'image de la caméra (114) restant verrouillés au moins jusqu'à ce que l'étape e) ait été exécutée, l'étape f) comprenant la comparaison des au moins deux images capturées dans les étapes b) et e) respectivement, au moins une d'une différence de couleur et d'une différence d'intensité étant détectée, **caractérisé en ce que** l'étape f) comprend :

la détermination d'une valeur de mesure relative $R_{rel}$ à partir d'une couleur ou d'une intensité du champ de test de la première image $I_{TF\_1}$ ou des deux, et d'une couleur et/ou d'une intensité du champ de test de la deuxième image $I_{TF\_2}$ en utilisant la fonction suivante :

$$R_{rel} = I_{TF\_2}/I_{TF\_1},$$

et/ou **en ce que**
l'étape f) comprend la détermination d'une valeur de mesure relative double $R_{twice\ rel}$ à partir d'une couleur et/ou d'une intensité du champ de test de la première image $I_{TF\ 1}$, d'une couleur et/ou d'une intensité du champ de test de la seconde image $I_{TF\ 2}$, d'une couleur et/ou d'une intensité d'une zone de référence sur la bandelette de test optique de la première image $I_{WF\ 1}$ et d'une couleur et/ou d'une intensité de la même zone de référence sur la bandelette de test optique de la seconde image $I_{WF\ 2}$, en utilisant les fonctions suivantes :

$$R_{twice\_rel} = (I_{TF\_2}/I_{WF\_2})/(I_{TF\_1}/I_{WF\_1}).$$

2. Procédé selon la revendication précédente, l'étape b) comprenant en outre la dérivation d'au moins un paramètre relatif à un ou plusieurs réglages d'acquisition d'image.

3. Procédé selon la revendication précédente, le paramètre relatif à un ou plusieurs réglages d'acquisition d'image étant stocké dans une base de données.

4. Procédé selon la revendication précédente, l'au moins un paramètre relatif au ou aux réglages d'acquisition d'image étant sélectionné dans le groupe constitué par : une vitesse d'obturation, spécifiquement un temps d'exposition ; une distance de mise au point ; un ajustement de couleur, tel qu'une saturation de couleur ; une correction de pixel, spécifiquement une réduction de bruit.

5. Procédé selon la revendication précédente, au moins l'un des au moins un paramètres relatifs au ou aux réglages d'acquisition d'image étant configuré pour être corrigé ou réglé par au moins un algorithme.

6. Procédé selon l'une quelconque des revendications précédentes, l'étape c) comprenant l'un ou les deux éléments suivants :

- inviter un utilisateur à appliquer l'échantillon de fluide corporel sur le champ de test (118) de la bandelette de test optique (116) ; ou
- inviter l'utilisateur à confirmer l'application de l'échantillon de fluide corporel sur le champ de test (118) de la bandelette de test optique (116).

7. Procédé selon l'une quelconque des revendications précédentes, le procédé comprenant en outre :
g) l'évaluation d'au moins une condition d'éclairage ambiant,
l'étape g) étant réalisée au moins une fois à un ou plusieurs des moments suivants :

- entre l'étape a) et l'étape b),
- entre l'étape b) et l'étape c),
- entre les étapes e) et f), ou
- après l'étape f).

8. Procédé selon la revendication précédente, l'étape g) comprenant en outre l'adaptation de l'au moins une condition d'éclairage ambiant en allumant ou en éteignant au moins une source d'éclairage (120) du dispositif mobile (112).

9. Programme informatique comprenant des instructions exécutables par ordinateur pour mettre en œuvre le procédé selon l'une quelconque des revendications précédentes lorsque le programme informatique est exécuté sur un ordinateur ou un réseau informatique.

10. Dispositif mobile (112) pour réaliser une mesure analytique, le dispositif mobile (112) ayant au moins une caméra (114), le dispositif mobile étant configuré pour réaliser au moins les étapes b), d), e) et f) du procédé de réalisation d'une mesure analytique selon l'une quelconque des revendications précédentes faisant référence à un procédé de réalisation d'une mesure analytique.

11. Dispositif mobile (112) selon la revendication précédente, le dispositif mobile (112) comprenant en outre au moins une source d'éclairage (120) configurée pour adapter au moins une condition d'éclairage ambiant.

12. Dispositif mobile (112) selon l'une quelconque des deux revendications précédentes, le dispositif mobile (112) comprenant au moins un processeur (122) étant programmé pour commander au moins une des étapes b), d), e) et f), et éventuellement c) et/ou g).

13. Kit (110) pour effectuer une mesure analytique, le kit (110) comprenant :

- au moins un dispositif mobile (112) selon l'une quelconque des revendications précédentes faisant référence à un dispositif mobile (112) ; et
- au moins une bandelette de test optique (116) comportant au moins un champ de test (118).

Fig. 1

```
┌─────────────┐
│     124     │
└──────┬──────┘
       │
       ▼
┌─────────────┐
│     126     │
└──────┬──────┘
       │
       ▼
┌─────────────┐
│     128     │
└──────┬──────┘
       │
       ▼
┌─────────────┐
│     130     │
└──────┬──────┘
       │
       ▼
┌─────────────┐
│     132     │
└──────┬──────┘
       │
       ▼
┌─────────────┐
│     134     │
└─────────────┘
```

Fig. 2 A

```
┌─────────────┐
│     124     │
└──────┬──────┘
       │
       ▼
┌─────────────┐
│     136     │
└──────┬──────┘
       │
       ▼
┌─────────────────┐
│       126       │
│  ┌───────────┐  │
│  │    138    │  │
│  └─────┬─────┘  │
│        ▼        │
│  ┌───────────┐  │
│  │    140    │  │
│  └───────────┘  │
└────────┬────────┘
         │
         ▼
┌─────────────────┐
│       128       │
└────────┬────────┘
         │
         ▼
┌─────────────────┐
│       130       │
└────────┬────────┘
         │
         ▼
┌─────────────────┐
│       132       │
└────────┬────────┘
         │
         ▼
┌─────────────────┐
│       134       │
│  ┌───────────┐  │
│  │    142    │  │
│  └───────────┘  │
└─────────────────┘
```

Fig. 2 B

Fig. 3 A

Fig. 3 B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0821234 A2
- EP 2916117 A1
- WO 2014037462 A1
- EP 1189064 A1
- EP 0953149 B1
- US 20150233899 A1
- US 20170098137 A1
- US 20130267032 A1
- WO 2017059103 A1

**Non-patent literature cited in the description**

- **J. HOENES et al.** The Technology Behind Glucose Meters: Test Strips. *Diabetes Technology & Therapeutics,* 2008, vol. 10 (1), S10-S26